# EUROPEAN PATENT APPLICATION

(11) **EP 1 382 601 A1**
(43) Date of publication of application: **21.01.2004**
(21) Application number: 02718608.9
(22) Date of filing: 18.04.2002
(51) Int. Cl.: C07D 301/19, C07D 303/04, C07D 301/32

(54) **PROCESS FOR PRODUCING PROPYLENE OXIDE**

(30) Priority: 27.04.2001 JP 2001132004
(71) Applicant: Sumitomo Chemical Company, Limited, Osaka-shi Osaka 541-8550 (JP)
(72) Inventor: OKU, Noriaki, Ichihara-shi, Chiba 290-0038 (JP); TSUJI, Junpei, Ichihara-shi, Chiba 299-0125 (JP)
(74) Representative: Henkel, Feiler, Hänzel
(86) International application number: PCT/JP2002/003848
(87) International publication number: WO 2002/088103

(57) **Abstract**

A process for producing propylene oxide, which comprises steps of reacting propylene with cumene hydroperoxide in the presence of a catalyst to obtain propylene oxide, and subjecting the reaction mixture obtained the above reaction step to distillation and recovering unreacted propylene from the top of a distillation column, wherein a bottom temperature of the distillation column is set at 200°C or lower.

## Description

### TECHNICAL FIELD

The present invention relates to a process for producing propylene oxide. More particularly, the present invention relates to a process for producing propylene oxide, which converts propylene into propylene oxide using cumene hydroperoxide as an oxygen carrier, and further , can be conducted under high yield.

### BACKGROUND ART

A process in which propylene is oxidized using ethylbenzene hydroperoxide as an oxygen carrier to give propylene oxide and styrene, is known as Halcon process. Further, when cumene hydroperoxide is used, cumyl alcohol together with propylene oxide is produced. Cumyl alcohol is converted into α-methylstyrene by dehydrogenation, or after cumyl alcohol is converted into cumene by hydrogenation, cumene can be recycled by converting into cumene hydroperoxide again by oxidation.

Though a concept of a process in which only propylene oxide is produced using cumene repeatedly, is described in Czechoslovak Patent No. CS 140,743, the process described in said patent does not contain precise descriptions concerning necessary steps exceptoxidationstep,epoxidationstep and hydrogenolysis step. Various problems arise in practical recycling of cumene and therefore the patent cannot be said as sufficient for industrial realization.

### DISCLOSURE OF THE INVENTION

Under such circumstances, a subject to be solved by the present invention is to provide a process for producing propylene oxide, which converts propylene into propylene oxide using cumene hydroperoxide as an oxygen carrier, can be conducted under high yield and is extremely advantageous from the viewpoint of industrial operation thereby.

Namely, the present invention relates to a process for producing propylene oxide, which comprises steps of:
reacting propylene with cumene hydroperoxide in the presence of a catalyst to obtain propylene oxide, and
subjecting a reaction mixture obtained in the above reaction to distillation and recovering unreacted propylene from the top of a distillation column, wherein a bottom temperature of the distillation column is set at 200°C or lower.

### BRIEF DESCRIPTION OF THE DRAWING

Fig. 1 shows a general flow of the producing process of the present invention.
Fig. 2 shows a general flow of the producing process of the present invention.

### (Explanation of symbols)

1. Cumene hydroperoxide
2. Propylene raw material
3. Reaction liquid
4. Reaction liquid after recovering propylene
5. Propylene recovered in a recovery step
6. Make-up propylene
7. Reaction liquid af ter a part of propylene has been recovered in the recovering step
8. Propylene recovered in recovery steps 1 and 2
9. Propylene recovered in the recovery step 2
10. Reaction liquid after propylene has been recovered in the recovery step 2
11. Propylene to be recycled to the recovery step 1 in propylene recovered in the recovery step 2.

### BEST MODE FOR CARRYING OUT THE INVENTION

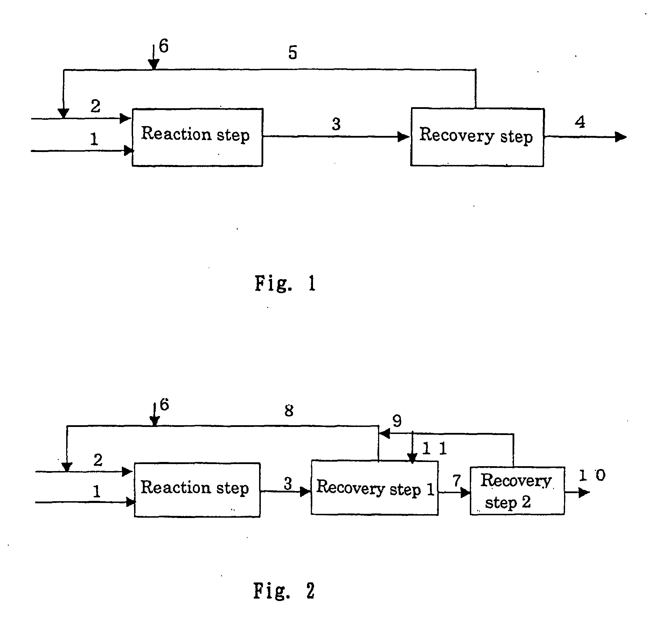

As a preferable mode for carrying out the present invention, one example of a general flow used for producing propylene is illustrated using Fig. 1.

A fresh cumene hydroperoxide solution through a line 1 and propylene through a line 2 are fed in the reaction step.

A reaction liquid discharged from the reaction step is supplied to the recovery step through a line 3, propylene is recycled through a line 5 to the reaction step after distilled for separation, and propylene consumed in the reaction step is supplied through a line 6. The reaction liquid containing propylene oxide after which propylene is recovered in the recovery step, is sent to the next step through a line 4.

The reaction step in the present invention means a step of reacting propylene with cumene hydroperoxide in the presence of a catalyst to produce propylene oxide.

In the present invention, propylene oxide is obtained by reacting cumene hydroperoxide obtained by auto-oxidation of cumene with air or air in which oxygen is concentrated, with propylene in the presence of a catalyst to bring propylene into epoxidation.

The epoxidation is preferably conducted in the presence of a catalyst containing a titanium-containing silicon oxide from the viewpoint that the desired product should be obtained under high yield and high selectivity. The catalyst is preferably a catalyst containing titanium chemically bonded to silicon oxide, so-called a titanium-silica catalyst. Examples may include products carrying a titanium compound on a silica carrier, products in which a titanium compound is compoundedwith a silicon oxide by a co-precipitation or sol-gel method, titanium-containing zeolite compounds and the like.

In the present invention, cumene hydroperoxide used as the raw material for the reaction step may be a dilute or thick purification or non-purification product.

The reaction step in the present invention can be conducted in a liquid phase using a solvent. The solvent is liquid under the reaction temperature and pressure, and preferably substantially inert to the reactants and the product. The solvent may be composed of a substance existing in a solution of the hydroperoxide used. When, for example, a cumene solution of cumene hydroperoxide is used as a raw material, it is also possible to use cumene as a solvent without adding a solvent in particular. Other useful solvents include aromatic monocyclic compounds (for example, benzene, toluene, chlorobenzene and o-dichlorobenzene), alkane (for example, octane, decane and dodecane) and the like. In the present invention, the reaction temperature in the reaction step is usually 0 to 200°C and preferably 25 to 200°C. The pressure is usually 100 to 20000 kPa and preferably 100 to 10000 kPa taking account of the reaction temperature and economical points.

The reaction step in the present invention can be advantageously carried out using a catalyst in the form of a slurry or a fixed-bed. The fixed-bed is preferred in the case of a large-scale industrial operation. In addition, the reaction can be carried out by a batch process, a semi-continuous process, a continuous process or the like. When a liquid containing the raw materials for reaction is passed through a fixed-bed, the catalyst is not contained at all or substantially in a liquid mixture discharged from a reaction zone.

In the reaction step in the present invention, when a fixed bed is used, a catalyst layer may be divided into multi layers and fresh cumene hydroperoxide and propylene may be fed divisionally to each of catalyst layers divided, further a reaction product discharged from an outlet of each of catalyst layers may be recycled to an inlet of each of the catalyst layers , respectively.

This method is effective for preventing a run-away caused by heat of the reaction and for carrying out stably the reaction under high yield.

In the present invention, the amount of propylene fed to the reaction step is usually 1 to 20 times by mole, preferably 5 to 20 times by mole per mole of fresh cumene hydroperoxide to be fed for epoxidation. To use an excess amount of propylene to fresh cumene hydroperoxide is effective for keeping a yield of produced propylene oxide at high yield.

The concentration of propylene to be supplied to the reaction step in the present invention is usually 70 % by weight or more, preferably 80 % by weight or more. As other impurities, it may contain components substantially inert to epoxidation such as propane, ethane, ethylene, methane, propylene oxide and the like.

The recovery step in the present invention, is a step of subjecting the reaction mixture obtained in the reaction step to distillation and recovering unreacted propylene from the top of a distillation column, and it is necessary that the operation bottom temperature of the distillation column is 200°C or lower. When the bottom temperature becomes higher than 200°C, not only propylene is lost but also a large amount of energy for distillation separation in a propylene oxide purification step as the next step, is required, because of formation of hydrocarbons of 6 carbon atoms through dimerization of unreacted propylene in the column. Further, in the reaction step, because water by dehydration of cumyl alcohol formed with propylene oxide, generates, a large amount of energy for distillation separation in a propylene oxide purification step as the next step is required similar to dimerized compounds of propylene. Furthermore, the generated water forms glycols through a reaction with propylene oxide, and therefore, decreases the yield of propylene oxide.

Moreover, loss of cumene leading to decrease of the yield, also generates through a conversion into heavy materials of unreacted cumene hydroperoxide and cumyl alcohol formed in the reaction step. From the above viewpoints, it is necessary to control the bottom temperature in the distillation operation in the recovery step within the range of the present invention. As a method of controlling the bottom temperature, there is, for example, a method of lowering the operation pressure, or a method of lowering a boiling point of the bottom by recovering a part of propylene to the bottom of the column. In a case that the temperature is controlled by lowering the operation pressure, when the bottom temperature is intently decreased to lower than 50°C, industrial operation becomes difficult because the pressure in the column must be high vacuum such as less than 0.07 MPa, whereby the condensation temperature of propylene recovered from the top of the column becomes to lower than-50 °C.

Therefore, when the operation pressure in the column is lowered thereby to control the bottom temperature, the bottom temperature is usually within a range of 50 to 200°C, preferably 80 to 200°C, more preferably 100 to 200°C. When the bottom temperature is lowered by recovering a part of propylene to the bottom of the column, the bottom temperature can be controlled to 200°C or lower by recovering propylene to the bottom of the column even if the operation pressure is 2.0 MPa or higher. Propylene collected from the bottom can be easily recovered.

For example, as shown in Fig. 2 , propylene can be repeatedly used with reduction of propylene loss by: subjecting a propylene oxide-containing liquid containing propylene to the recovery step 2 as a distillation column through a line 7; condensing propylene obtained from the top of the column to liquefy propylene followed by raising the pressure with a pump or raising the pressure of propylene obtained from the top of the column with a compressor; and recycling propylene to the recovery step through a line 11 or recycling directly to the reaction step through a line 9. As described herein, it is effective for preventing reduction of a propylene yield to recover propylene with two or more of distillation columns. When the bottom temperature is lowered by recycling a part of propylene to the bottom of the column, the effect becomes large in proportion to the concentration of propylene in the bottom of the column. However, when the amount recovered in the bottom of propylene becomes large, increases of the power and energy for recovering propylene to the bottom, are caused. Therefore, the range is usually 50 % by weight or less, preferably 0.1 to 40 % by weight, more preferably 0.1 to 30 % by weight.

### EXAMPLE

### Examples 1 to 4

A reaction liquid in a reaction step as a material corresponding to a bottom liquid, was charged into a pressure-proof 150 cc-container, then propylene was chargedwith its pressure to dissolve it in the liquid to prepare a mixed solution composed of 0 . 2 % by weight of cumene hydroperoxide, 71.2 % by weight of cumene and 28.6 % by weight of propylene and to heat it at a temperature shown in Table 1 for 0.5 hour in the closed system. After 0.5 hour, the amount of propylene dimers formed in the liquid was measured by a gas chromatograph. The measurement results are shown in Table 1.

**Table 1**

| | Example 1 | Example 2 | Example 3 | Example 4 |
|---|---|---|---|---|
| Temperature (°C) | 113 | 132 | 152 | 171 |
| Propylene dimers formed (% by weight) | Trace | Trace | 0.0013 | 0.0047 |

From these results, it is found that, also in the bottom liquid, the formed amount of propylene dimers is extremely small within the range of the present invention.

### INDUSTRIAL APPLICABILITY

As described above, according to the present invention, there can be provided a process for producing propylene oxide, having excellent characteristics in which the process converts propylene into propylene oxide using cumene hydroperoxide as an oxygen carrier, can be conducted under high yield and further can reduce a load in a purification step of propylene oxide as a product.

## Claims

1. A process for producing propylene oxide, which comprises steps of:
reacting propylene with cumene hydroperoxide in the presence of a catalyst to obtain propylene oxide, and
subjecting a reaction mixture obtained the above reaction step to distillation and recovering unreacted propylene from the top of a distillation column, wherein a bottom temperature of the distillation column is set at 200°C or lower.

2. The process for producing propylene oxide according to claim 1, wherein the bottom temperature of the distillation column is 80 to 200 °C.

3. The process for producing propylene oxide according to claim 1, wherein the concentration of propylene in the bottom of the column is 50 % by weight or less.

4. The process for producing propylene oxide according to claim 1, wherein the concentration of propylene in the bottom of the column is 0.1 to 40 % by weight.
